# EUROPEAN PATENT SPECIFICATION

(11) **EP 4 259 152 B1**
(45) Date of publication and mention of the grant of the patent: **19.06.2024**
(21) Application number: 21840712.0
(22) Date of filing: 13.12.2021
(51) Int. Cl.: A61K 31/53, A61P 35/00, C07D 487/04

(54) **CEMTIRESTAT DISULFIDE, PRODRUG OF ALDO-KETO REDUCTASE INHIBITOR, PREPARATION, PHARMACEUTICAL COMPOSITION AND USE THEREOF**
CEMTIRESTATDISULFID, ALDO-KETO-REDUKTASE-VORLÄUFER, HERSTELLUNG, PHARMAZEUTISCHE ZUSAMMENSETZUNG UND VERWENDUNG DAVON
DISULFURE DE CEMTIRESTAT, PROMÉDICAMENT D'INHIBITEUR D'ALDO-CÉTO RÉDUCTASE, PRÉPARATION, COMPOSITION PHARMACEUTIQUE ET UTILISATION DE CELUI-CI

(30) Priority: 14.12.2020 SK 500742020
(43) Date of publication of application: 18.10.2023
(73) Proprietor: Centrum Experimentálnej Medicíny SAV, v.v.i., 841 04 Bratislava (SK); Univerzita Komenskeho V Bratislave, 814 99 Bratislava - mestska cast Stare Mesto (SK)
(72) Inventor: STEFEK, Milan, 900 68 Plavecký Stvrtok (SK); KOVÁCIKOVÁ, Lucia, 831 04 Bratislava (SK); SOLTÉSOVÁ-PRNOVÁ, Marta, 841 01 Bratislava (SK); ADDOVÁ, Gabriela, 851 04 Bratislava (SK); BOHÁC, Andrej, 851 04 Bratislava (SK)
(74) Representative: Majlingová, Zuzana
(86) International application number: PCT/SK2021/050015
(87) International publication number: WO 2022/132058

(56) References cited:
- WO-A1-2015/057175
- WO-A1-2017/064675
- STEFEK MILAN ET AL: "Identification of Novel Aldose Reductase Inhibitors Based on Carboxymethylated Mercaptotriazinoindole Scaffold", JOURNAL OF MEDICINAL CHEMISTRY, vol. 58, no. 6, 26 March 2015 (2015-03-26) , pages 2649-2657, XP055889575, US ISSN: 0022-2623, DOI: 10.1021/jm5015814 Retrieved from the Internet: URL:https://pubs.acs.org/doi/pdf/10.1021/j m5015814>
- ROUT SUBHASHREE ET AL: "In silico study of M18 aspartyl amino peptidase (M18AAP) ofPlasmodium vivaxas an antimalarial drug target", BIOORGANIC, vol. 27, no. 12, 22 March 2019 (2019-03-22), pages 2553-2571, XP085695693, ISSN: 0968-0896, DOI: 10.1016/J.BMC.2019.03.039

## Description

### Technical field

The present invention relates to a pharmaceutically usable prodrug of the pharmacologically active compound cemtirestat, which is an inhibitor of aldo-keto reductases, especially aldose reductase (AR, AKR1B1), method for its preparation, a pharmaceutical composition containing it and said prodrug of formula (I) for use in the treatment of human and veterinary diseases.

### Background Art

Aldo-keto reductases are NAD(P)H-dependent oxidoreductases, which are best characterized as glucose lowering agents. They are involved in the pathophysiology of diabetic complications. These enzymes also metabolize lipid peroxidation products, thus contributing to an inflammatory response in certain circumstances.

Aldose reductase (AR, AKR1B1), in addition to being involved in diabetic complications through glucose reduction, effectively reduces aldehydes formed by lipid peroxidation as well as their conjugates with glutathione (Ramana, BioMolConcepts 2: 103-114, 2011). Aldehydes from lipid peroxidation such as 4-hydroxy-trans-2-nonenal (systemic name trans 4-hydroxynon-2-en-1-al) (HNE) and glutathione conjugates thereof (e.g. GS-HNE) are effectively reduced by AR to the corresponding alcohols, DHN (1,4-dihydroxynonene, systemic name trans non-2-ene-1,4-diol) and GS-DHN (glutathione-1,4-dihydroxynon-2-ene), which mediate inflammatory signals in the body. The reduced GS-DHN conjugate is thought to be a signaling intermediate in the transmission of cellular signals initiated by reactive oxygen species, which may ultimately lead to an inflammatory response (Srivastava et al.; ChemBiolInteraction 191: 330-338, 2011; Balestri et al.; Antioxidants (Basel) 8 (10). pii: E502, 2019; Srivastava et al.; Free Radic. Biol. Med 29: 642-651, 2000; Shoeb et al.; Curr. Med Chem. 21: 230-237, 2014). In cell and animal models, AR inhibition effectively eliminated inflammatory signals induced by cytokines, growth factors, endotoxins, high glucose, allergens, and autoimmune responses (Ramana and Srivastava, Int. J. Biochem. Cell Biol. 42: 17-20, 2010).

It is very well documented that chronic inflammation is associated with cancer progression (Solinas et al. Cancer Metastasis Rev. 29, 243-248, 2010; Khayami et al.; J. Cell. Mol. Med. 2020; 10.1111 / jcmm.15581). Epidemiological studies suggest that > 25 % of all cancers are closely related to chronic infection and chronic inflammation (Vendramini-Costa and Carvalho, CurrPharm Des. 18: 3831-52, 2012). Increased expression of aldo-keto reductases has been reported in the lung, breast, prostate, cervix, testes, and colon tumors (Liu et al.; Recent Pat Anticancer DrugDiscov. 4: 246-53, 2009; Laffin and Petrash, Front Pharmacol. 3: 104, 2012; Terzig et al Gastroenterology 138: 2101-2114, 2010; Reddy et al.; Breast 31: 137-143, 2017). In addition, increased expression of AKR1B1 in various cancers causes resistance to doxorubicin, which is explained by increased AKR1B1-mediated detoxification of doxorubicin by carbonyl reduction (Lee et al.; Anticancer Drugs 2: 129-32, 2001). Several studies have suggested that inhibition of AKR1B1 as adjuvant therapy increased tumor sensitivity to anti-cancer therapy or alleviated adverse side effects (Banala et al.; BiomaterSci. 7: 2889, 2019; Khayami et al.; J. Cell Mol. Med. 2020; 10.1111). jcmm.15581).

In addition to AKR1B1, the related enzyme AKR1B10 is involved in several types of cancer (Penning, ClinCancerRes. 11: 1687-90, 2005; Fukumoto et al.; ClinCancerRes. 11: 1776-1785, 2005; Yan et al.; Int. J. Cancer 121: 2301-6, 2007; Zhao et al.; Eur. J. Med Chem. 45 (9): 4354-7, 2010; Matsunaga et al.; Front Pharmacol. 3: 5.2012; Laffin and Petrash, Front Pharmacol. 3: 104, 2012; Liu et al.; Biochem J. 442: 273-82, 2012). AKR1B10 is a 36-kDa cytosolic reductase that is similar to AKR1B1 by amino acid sequence (71% identity) as well as by a tertiary structure with a "(a / β) 8 barrel" topology. Like AKR1B1, the AKR1B10 enzyme reduces a variety of aromatic and aliphatic aldehydes, dicarbonyl compounds, and some carbonyl-containing drugs using NADPH as a coenzyme. AKR1B10 is normally produced in the gastrointestinal tract. Overexpression occurs in many tumors such as hepatic, lung and breast carcinoma.

AKR1B10 may be involved in carcinoma development by multiple mechanisms and may be a useful biomarker for cancer diagnosis and a potential therapeutic target (Huang et al.; Recent Pat Anticancer Drug Discov. 11: 184-196, 2016). Silencing of AKR1B10 expression resulted in inhibition of colorectal carcinoma cell proliferation (Yan et al. Int. J. Cancer 121: 2301-6 2007).

The role of aldo-keto reductases in the etiology of colorectal carcinoma has been confirmed (Tammali et al.; CancerRes. 66, 9705-9713, 2006; Tammali et al.; CancerLett 252, 299-306, 2007). Analysis of colorectal carcinoma cell lines indicated that high expression levels of AKR1B1 and AKR1B10 correlated significantly with disease progression (Taskoparan et al.; CellOncol. (Dordr). 40: 563-578, 2017). Aldose reductase inhibition thus appears to be a promising therapeutic target in the treatment of colorectal carcinoma (Grewal et al.; Mini Rev. Med Chem. 16, 120-162, 2016; Saxena, et al. CancerLett. 355, 141-147, 2014; Saxena et al., Eur. J. Cancer. 49 (15): 3311-9, 2013; Tammali et al.; Carcinogenesis, 32: 1259-1267, 2011; Shoeb et al.; FreeRadic. Biol. Med. 63: 280-290, 2013 , Tammali et al.; Carcinogenesis 8: 1259-67, 2011; Tammali et al.; CurrCancerDrugTargets. 11: 560-71, 2011; Ramana et al.; Mol CancerTher., 9: 813-24, 2010; Shukla et al., CancerLett. 28; 411: 57-63, 2017).

In HeLa cell culture studies (Ji et al.; Mol. Biol. Rep. 47: 6091-6103, 2020), the aldose reductase inhibitor epalrestat inhibited tumor progression by inhibiting AKR1B1 and was proposed as a new drug for treatment of cervical cancer. In a study in SCID mice (Wu X, et al.; J. Exp. Med. 214: 1065-1079, 2017), epalrestat dramatically inhibited the progression of basal breast cancer.

The aldose reductase inhibitor fidarestat *in vitro* and *in vivo* increased the sensitivity of cancer cells to oxidative stress and suppressed their growth (Shukla et al.; CancerLett 411: 57, 2017), inhibited angiogenesis and suppressed tumor proliferation (Tammali et al.; Angiogenesis 14: 209, 2011 ).

U.S. Patent No. 20110925666 relates to methods of treating lung, breast and prostate cancer and suppressing metastases associated with colon cancer using aldose reductase inhibitors.

The authors of Banala et al. (BiomaterSci. 7: 2889, 2019) developed a micellar carrier of aldose reductase inhibitor epalrestat (EPR) based on a disulfide (systemic name "disulfane") conjugate with tocopherol-polyethylene glycol-succinate (TPGS). In a reducing environment, controlled release of epalrestat occurred by cleavage of the disulfide (systemic name "disulfane") bond of the EPR-S-S-TPGS conjugate.

Patent SK285588B6 describes biologically acceptable disulfides of thioctic acid, tetranorlipoic acid, bisnorlipoic acid and 8-hydroxy-bisnorlipoic acid in admixture with essential fatty acids for the treatment and prevention of diabetes, insulin resistance or complications in diabetes.

WO2019040825 and WO2017064675 describe the use of a chemotherapeutic agent in a disulfide-based conjugate for the treatment of cancer.

U.S. Patent No. 20010036926 describes labile disulfide-bonded substances which are activated under physiological conditions by the action of free thiols.

The above findings suggest that AKR1B1 and AKR1B10 inhibitors are expected to be useful in cancer treatment as new therapeutics. AKR1B1 inhibitors, such as acetic acid derivatives (epalrestat, tolrestat, zenarestat), spirohydantoins (sorbinil) or succinimide compounds (ranirestat), have been investigated mainly for their role in the prevention of diabetic complications (Hotta, BiomedPharmacother. 5, 244-250 1995. ; Costantino et al.; Expert OpinTherPatents. 10, 1245-1262, 2000; Miyamoto, Expert OpinTherPatents. 2002, 12, 621-631 2002; Srivastava etal.; Endocr Rev. 26, 380-392 2005). In the search for better AKR inhibitors, interest has shifted in recent years to new chemotypes (Alexiou et al.; Curr. Med Chem. 16: 734-52, 2009; Chatzopoulou et al.; Expert OpinTher Pat. 11: 1303-23, 2012).

Although a number of aldo-keto reductase inhibitors have been extensively studied, none of them have demonstrated sufficient efficacy in human clinical trials without adverse side effects. Thus, there is still a need to develop new, effective and safe inhibitors of the aldo-keto reductases AKR1B1 and AKR1B10 as potential drugs for diabetic complications, inflammatory diseases and cancer.

3-Mercapto-5*H-*1,2,4-triazino[5,6-*b*]indole-5-acetic acid (cemtirestat of formula **II**, systemic name 2-(3-thioxo-2,3-dihydro-5*H-*[1,2,4]triazino[5,6-*b*]indol-5-yl)acetic acid, has been proposed and patented as a highly potent and selective aldose reductase inhibitor with antioxidant properties (Stefek et al.; Patent SK288508B6; Stefek et al. , J. Med Chem. 58: 2649-57, 2015; Prnova et al.; RedoxRep. 20: 282-8 2015; Soltesova Prnova et al.; Physiol. Res. 64: 587-91, 2015; Stefek et al.; IJASEAT 4:41 -44, 2016; Zhan et al.; J. Biomol. Struct. Dyn. 37: 1724-1735, 2018, Soltesova Prnova et al.; Neuroscience 443: 206-217, 2020; Valachova et al.; Int J Mol Sci: E5609, 2020). The ability of cemtirestat of formula **II** to alleviate the symptoms of peripheral neuropathy in ZDF and STZ-induced diabetic rats has recently been described (Soltesova Prnova et al.; NeurochemRes. 44: 1056-1064, 2019; Prnova et al.; Naunyn Schmiedebergs Arch Pharmacol. 393:651-661 2020). Extremely low cytotoxicity of cemtirestat of formula **II** *in vitro* and *in vivo* was proved and in addition no significant changes were observed in Wistar rats in a 120-day toxicology test (Soltesova Prnova et al.; Interdiscip. Toxicol. 12: 120-128 2019).

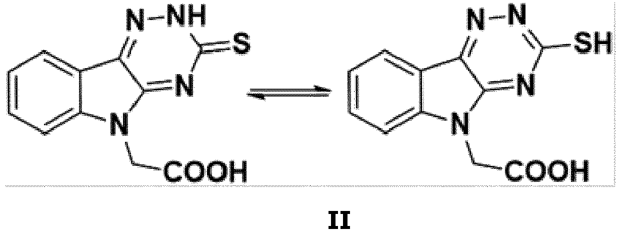

### Two tautomeric forms of cemtirestat of formula II

Although cemtirestat of formula **II** shows effective inhibition of the aldo-keto reductases AKR1B1 and AKR1B10, characterized by IC50 values = 0.48 ± 0.29, 3.69 ± 0.53 µM, respectively (Stefek et al.; J. Med Chem. 58: 2649-57, 2015), it would be advantageous to improve its pharmacokinetic properties for both oral and parenteral administration, and such desirable improvements include, in particular, improved absorption from the gastrointestinal tract, prolongation of the duration of action of the active compound and in particular targeted distribution to cancer cells. Oral and parenteral administration of active aldo-keto reductase inhibitors can lead to undesirable effects due to high local concentrations in healthy tissues such as hepatotoxicity, indigestion, renal, sperm and other disorders.

The invention proposes to solve these problems by using a therapeutic strategy based on the administration of a prodrug of an active drug. In this prodrug-based strategy, the active drug is released upon chemical or metabolic activation of the inactive prodrug at the site of desired effect. This approach can significantly increase the drug availability at the site of action and reduce toxicity compared to direct drug administration.

Cemtirestat disulfide of formula **I** was synthesized and tested to obtain a potent aldose reductase inhibitor. Given the key role of the aldo-keto reductases AKR1B1 and AKR1B10 in the etiology of several chronic inflammatory cancer types, as discussed above, we hypothesized that the release of cemtirestat of formula **II** within tumor cells by reducing its precursor with glutathione GSH would increase efficacy and drug action. This concept is based on the fact that reduced glutathione levels in tumors are 10-1000-fold higher compared to healthy tissues or the extracellular environment (Saito et al.; Adv. Drug Deliv. Rev. 55: 199, 2003; Wang et al.; Current Organic Chemistry 20 : 1477, 2016; Turell et al.; Free Radic. Biol. Med. 65: 244, 2013). The disulfide bond is expected to remain stable in the blood circulation as it cleaves upon entry into tumor tissue due to the high level of GSH in the tumor cells. A prodrug strategy based on the use of redox-sensitive disulfides is widely developed, especially in cancer therapy as a means of targeted distribution of chemotherapeutics to cancer cells (Meng et al.; Biomaterials30: 2180, 2009; Yang et al.; J. Phys. Chem. B118: 12311, 2014 Brülisauer et al.; J. Control Release 195: 147, 2014; Li et al.; Asian J. Pharm. Sci. 15: 311, 2020).

To our knowledge, this is the first example of a disulfide-based prodrug of aldose reductase inhibitor. Aldose reductase inhibitor prodrugs patented and published to date are based on esters (Bruno et al.; Bioorg. Med. Chem. 10: 1077, 2002; Da Settimo et al.; J. Med. Chem. 46: 1419-28, 2003; Da Settimo et al. al., Med. Chem. 48: 6897-907, 2005; Rakowitz et al.; Eur. J. PharmSci. 15: 11-20, 2002; Sunkara et al.; J. Pharm. Pharmacol. 52: 1113, 2000) or amides (Wrobel et al. J. Med. Chem. 34: 2504, 1991).

WO 2015/057175 relates to the use of 5-carboxymethyl-3-mercapto-1,2,4-triazino-[5,6-b]indoles and their pharmaceutically acceptable salts hydrates and solvates thereof for the use in treatment, control and prevention of human and veterinary diseases in which activities of aldo- keto reductases AKR1B1 and AKR1B10 are key etiological factors for their development and progress such as the development of diabetic complications (macro-, microangiopathy, atherosclerosis, retinopathy, cataracts, nephropathy, neuropathy, bone mass loss and atherosclerosis), inflammatory diseases (uveitis, sepsis, periodontitis, asthma and colorectal cancer), abnormal proliferation of vascular smooth muscle cells in atherosclerosis and restenosis, lung carcinoma in smokers, and several types of cancer (lung, breast, hepatic, prostate, pancreatic, endometrial cancer, cervical cancer, and cervical adenocarcinoma), diseases of the female reproductive system (menstrual disorders and fertility problems), timing of parturition, mood disorders, psychiatric and neurological diseases. This document relates to pharmaceutical compositions comprising effective amount of 5-carboxymethyl-3-mercapto-1,2,4-triazino-[5,6-b]indoles and a pharmaceutically acceptable carrier for the use in treatment, control and prevention of human and veterinary diseases.

### Summary of the Invention

The present invention provides the compound cemtirestat disulfide 2,2'-(disulfanediylbis(5*H*-1,2,4]triazino[5,6-*b*]indole-3,5-diyl))diacetic acid of formula **I**: 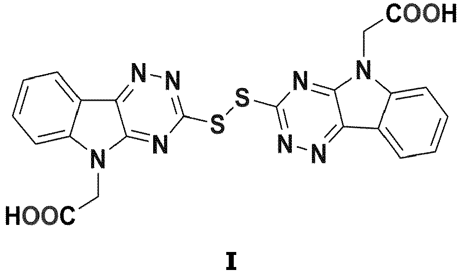 or a pharmaceutically acceptable form thereof: mono or dibasic salt, mono- or diesters, amides, or combinations thereof.

In addition to the structure of cemtirestat disulfide of the formula **I**, the invention also relates to a process for its preparation which comprises the following steps:
- treatment of the compound cemtirestat of formula **II** with a mild oxidizing agent NaNO₂ in acetic acid 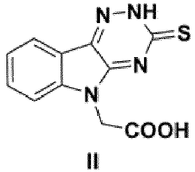
   at room temperature;
   wherein the product of formula **I** already precipitates from the reaction mixture during the reaction;
- filtration, washing the product with ice water and cold methanol;
- drying the product of formula **I** by means of a gradual vacuum obtained by a water pump and subsequently by an oil pump from 3000 Pa to 0.1 Pa.

In one embodiment of the invention, the compound cemtirestat disulfide of formula **I** or a pharmaceutically acceptable form thereof as mentioned above is for use as a medicament.

In another embodiment of the invention, the compound cemtirestat disulfide of formula **I** or a pharmaceutically acceptable form thereof as mentioned above, is for use in the treatment of conditions where inhibition of AKR1B1 and/or AKR1B10 aldo-keto reductases in the cells/tissues with high GSH (reduced glutathione) is desired.

In another embodiment of the invention, the compound cemtirestat disulfide of formula **I** or a pharmaceutically acceptable form thereof is used for the treatment of cancer originating from chronic inflammation, namely colon and rectal cancer, lung, breast, liver, pancreas, prostate, endometrial and cervix cancer.

In another embodiment of the invention, the compound cemtirestat disulfide of formula **I** or a pharmaceutically acceptable form thereof is for use in the treatment of cancer as an adjuvant therapeutic in combination with clinically used chemotherapeutics which are substrates of aldo-keto reductases such as doxorubicin and daunorubicin.

In yet another embodiment of the invention, the pharmaceutical composition comprising as active ingredient the compound cemtirestat disulfide of formula **I** or a pharmaceutically acceptable form thereof as mentioned above is in admixture with a pharmaceutically acceptable agent, diluent or carrier (excipient).

The invention also relates to a compound of formula **I** for use as a medicament as defined.

### Preparation

The compound of formula **I** of the present invention can be prepared by a number of methods well known to those skilled in the art of organic synthesis, including the methods described below or variations thereof. The methods described are preferred, but possible methods of preparation are not limited to these.

In general, symmetrical aromatic disulfides (systemic name disulfanes) can be prepared by oxidation of the appropriate aromatic thiols according to the reaction outlined in Scheme 1, according to procedures well known to those skilled in the art. Peroxydisulfates, e.g. (NH₄)₂S₂O₈ or K₂S₂O₈, can be used as oxidizing agents, at elevated temperature in acetonitrile, iodine, H₂O₂ or NaNO₂ at room temperature in ethanol or in weak acid or NaIO₄ in the solid state (Montazerozohori, et al., Molecules 12, 694-702, 2007; Ramadas et al., Organic Preparations and Procedures Int. 28, 352-355, 1996; Phakdeeyothin and Yotphan, Org. & Biomol. Chem. 17, 6432-40, 2019; Firouzabadi et al.; Synth. Comm. 28, 1179-87, 1998; Parida et al.; Chem. Select 1, 490-4, 2016; Carbonnel et al.; Chem. Comm. 53, 5706-9, 2017; Owen et al.; US6566384, 2003; Teplyakov et al.; Org. Lett. 15: 4038-41, 2013; Branc 2011, et al.; PCT Int. Appl. 209125191, 2009; Rubino, et al.; ChemMedChem. 6, 1258-68).

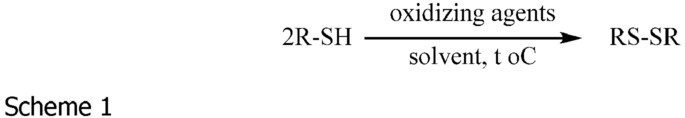

For example, the well-proven procedure used for the preparation of compound of formula **I,** shown in Scheme 2, can be used under conditions used by other authors on different type of substrate (Abazid, et al.; Phosphorus, Sulfur, and Silicon and the Related Elements 1994, 88 (1-4), 195-206) in the synthesis of various disulfides.

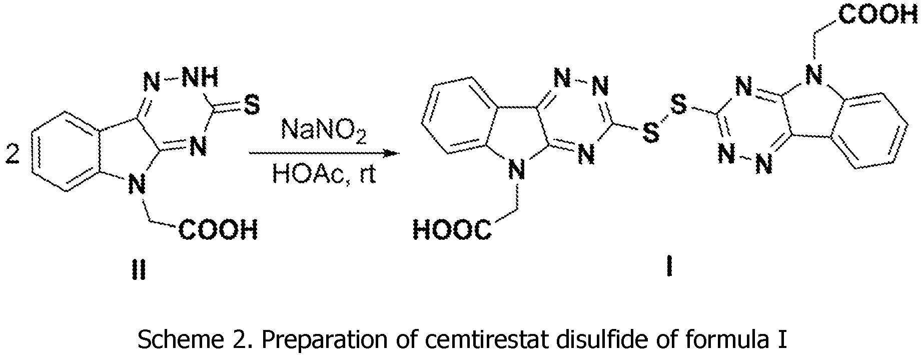

Cemtirestat of formula **II**, a starting material for the synthesis of a compound of formula **I,** can be prepared by synthetic procedures well known to those skilled in the art, that are described in standard works such as Romanchick and Joullie, Heterocycles 9, 1631, 1978; Neunhoeffer, H., Wiley, P. E. in Chem. Heterocycl. Compd.; Wiley-Interscience: New York, 1978, 33, 749; El Ashry, E. S. H.; Rashed, N.; Taha, M. in Advances in Heterocyclic Chemistry; Katritzky, A.R. Ed.; Academic Press: New York, 59 (1994).

For the preparation of cemtirestat of formula **II**, our previously published procedure Hlaváč et al.; *J. Med. Chem.* 63: 369-381, 2020 using starting isatin (**a**, Scheme 3) can be used. The reaction was performed by deprotonation by calcium hydride, where compound **a** was alkylated to **b** in an excess of ethyl chloroacetate in DMF by stirring at 100 ° C within 5 hours. Subsequent condensation with thiosemicarbazide in DMF at 100 °C, semicarbazone **c** was obtained. Finally, intermediate **c** was refluxed in aqueous potassium carbonate solution within two days, yielding cemtirestat of formula **II** in a total yield of 39%.

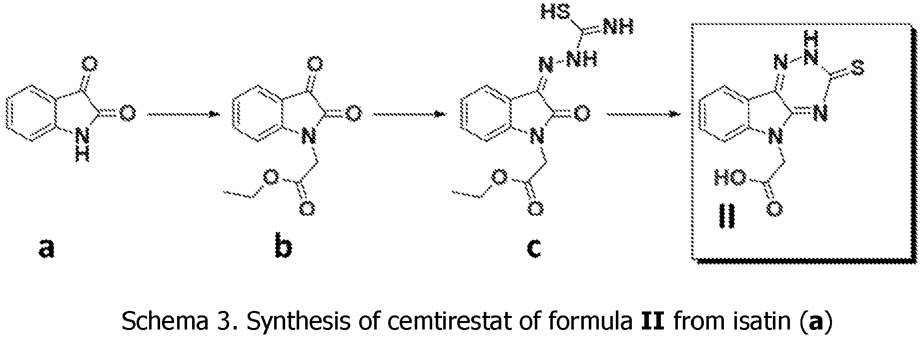

### Use in medicine and pharmacy

The invention also relates to the use of disulfide of formula **I,** which consists in that this substance is reductively metabolized in the body to form two molecules of cemtirestat of formula **II** which have pharmacological activity. Therefore, it is indicated as a pharmaceutical and in particular as a prodrug for the active compound of formula **II**.

Although Compound of formula **I** of the invention does not interact with aldose reductase (AKR1B1) and other aldo-keto reductases *per se*, it is metabolized in tissues characterized by increased reduction potential, such as tumor cells, to cemtirestat of formula **II**, which is a potent inhibitor of aldo-keto reductases AKR1B1 and AKR110. Statement "Compound of formula **I** according to the invention does not interact with the aldo-keto reductases AKR1B1 and AKR1B10 *per se* "is understood that compound of formula **I** has an IC50 value for the inhibition of AKR1B1 or AKR1B10 higher than 100 µmol/l.

Thus, compound of formula **I** of the invention is expected to be useful in conditions characterized by increased reduction potential where inhibition of AKR1B1 or AKR1B10 is required, such as tumor cells. These are characterized by significantly increased levels of reduced glutathione (GSH) compared to the physiologically healthy cells and especially compared to the extracellular space (gastrointestinal tract, central blood compartment, etc.). Conditions in which compound of formula **I** of the invention may find use include cancer of the colon, lung, breast, liver, prostate, pancreas, endometrium, cervix, etc., in a therapeutic and/or prophylactic regimen.

Another aspect of the invention is a method of treating a condition where inhibition of AKR1B1 or AKR1B10 aldo-keto reductases is targeted to tissues characterized by increased reduction potential, such as tumor cells characterized by significantly increased levels of reduced glutathione (GSH) compared to the healthy cells or the extracellular space (gastrointestinal tract, central blood compartment, etc.). This method comprises administering to a subject suffering from or susceptible to such a condition a therapeutically effective amount of a disulfide of formula **I,** or a pharmaceutically acceptable form thereof.

Another aspect of the invention is the use of disulfide of formula **I** for the treatment of cancer as an adjuvant therapeutic in combination with clinically used chemotherapeutics that are substrates of aldo-keto reductases such as doxorubicin and daunorubicin, or similar types of chemotherapeutics. This method comprises administering a therapeutically effective amount of disulfide of formula **I,** or a pharmaceutically acceptable form thereof, as described above, in combination with a therapeutic dose of a clinically used chemotherapeutic such as doxorubicin, daunorubicin, etc., to a subject suffering from or susceptible to such a condition.

The disulfide of formula **I** according to the invention is an inactive compound against AKR1B1 and other aldo-keto reductases. Thus, the compound of formula **I** remains inactive in healthy cells and in the extracellular space (gastrointestinal tract, central blood compartment, etc.), which are characterized by a reduced reduction potential (low GSH level), and thus avoids the potential side effects known from orally administered active aldose reductase inhibitors such as hepatotoxicity, indigestion, renal, sperm and other disorders.

The advantage of disulfide therapy of formula **I** over cemtirestat of formula **II** or other aldose reductase inhibitors is the targeted distribution of the active drug to tumor cells, which can result in higher efficacy and fewer side effects compared to cemtirestat of formula **II**.

The disulfide of formula **I** according to the invention may also have the advantage that in the acidic environment of the tumors this compound is better absorbed compared to cemtirestat of formula **II** alone, as documented in Example 3 describing an almost 4-fold higher water/octanol distribution ratio for compound of formula **I** compared to cemtirestat of formula **II**, in an acidic environment at pH 4.6.

Disulfide of formula **I,** according to the invention, may also have the advantage that, due to its molecular symmetry, it provides two molecules of the effective AR inhibitor cemtirestat of formula **II** at the same time, and thus the treatment can be even more effective.

Disulfide of formula **I,** according to the invention, may also have other useful pharmacological properties which are more advantageous than known aldo-keto reductase inhibitors which are active *per se*.

Disulfide of formula **I** of the invention will normally be administered orally, buccally, rectally, dermally, nasally, tracheally, bronchially, or by any other parenteral route or by inhalation, or in the form of pharmaceutically acceptable non-toxic organic or inorganic salts, or pharmaceutically acceptable forms of compound of formula **I** as defined above. Depending on the disease and the patient to be treated and the routes of administration, the compositions may be administered in different doses.

The invention also relates to pharmaceutical compositions comprising a compound of formula **I** as defined or a pharmaceutically acceptable form thereof as defined above in admixture with a pharmaceutically acceptable agent, stabilizer, diluent or carrier.

### Brief description of the drawings

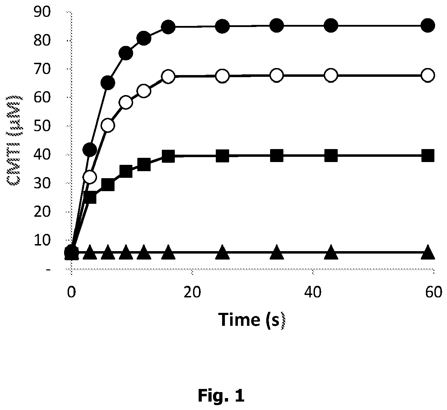

FIG. 1. Disulfide of formula **I** cleavage kinetics due to increasing concentrations of GSH to form cemtirestat of formula **II** (CMTI). Typical results from individual experiments are given. Disulfide of formula **I,** 50 µM; GSH 0 (▲); GSH 25 µM (▪); GSH 50 (M (∘); GSH 100 µM (•); experiments were performed uniformly in 20 mM phosphate buffer at pH 7.4 and 37 °C.

### Examples of embodiment

The invention is illustrated by the following examples, which present the preparation of disulfide of formula **I,** the measurement of the reduction kinetics of compound of formula **I** in the presence of various concentrations of GSH, the measurement of aldose reductase (AKR1B1) inhibition by disulfide of formula **I,** and the determination of the water/octan-1-ol distribution ratio. The examples given for disulfide do not limit the scope of the claims. It will be apparent to those skilled in the art that many modifications in terms of materials and methods may be practiced without departing from the spirit and scope of the invention.

### General experimental procedures

¹H-NMR and ¹³C-NMR measurements were performed on a Varian Gemini spectrometer (300 MHz or 600 MHz for ¹H and 75 MHz or 150 MHz for ¹³C-NMR) in CDCl₃ or DMSO-d₆. Chemical shifts are given in ppm with respect to the selected TMS standard and interaction constants *J* in Hz. IR spectra were measured on an Agilent Technologies Cary 630 FTIR in the range 650 - 4002 cm⁻¹ in a solid phase on a diamond detector without solvent and reference to the air. UV spectra were recorded on the Agilent Technologies Cary 8454 UV-Vis instrument. Liquid chromatography with mass spectrometry (LC-MS) analysis was performed on an Agilent Technologies 1200 Series instrument equipped with an Agilent Technologies 6100 Quadrupole mass spectrometer using electrospray ionization (ESI). Melting points were determined using a Büchi Melting Point M-565 and were not corrected. Elemental analysis (C, H, N) were performed using a Carlo Erba Strumentazione Model 1106.

### Preparation of the starting material

Cemtirestat of formula **II** (2-(3-thioxo-2,3-dihydro-5*H-*[1,2,4]triazino[5,6-*b*]indol-5-yl)acetic acid) was prepared according to the methods described in our publication Hlaváč et al.; *J. Med. Chem.* 63: 369-381, 2020.

### Example 1

### Syntthesis of disulfide of formula I - 2,2'-(disulfanediylbis(5H-[1,2,4]triazino[5,6-b]indole-3,5-diyl))diacetic acid

To a solution of 200 mg (0.768 mmol, 1.00 mol eq) of cemtirestat of formula **II** in 20 mL of glacial acetic acid, an excess of anhydrous sodium nitrite 138 mg (2.000 mmol, 2.60 mol eq) was added by stirring at room temperature. During the addition of NaNO₂, the reaction mixture changed its colour from yellow to brick red, releasing red nitrogen oxide containing fumes. A solid material precipitated out of the reaction mixture within ten minutes. The mixture was allowed to stand for another 1 hour. Product of formula **I** was isolated from the reaction mixture by filtration. The precipitate on the filter was washed with ice water and afterwards by ice-cold CH₃OH. The solid residue was dried in vacuo to give 163 mg (0.314 mmol, 82 %) of disulfide of formula **I** as a pale brown solid. M.p.: 220 - 250 °C (dec);
¹H-NMR (600 MHz, DMSO-*d*₆): *δ*13.23 (br, 2H, 2 x -COOH), 8.06 (dd, 2H, *J*(8,9) = 7.6 Hz, *J*(7,9) = 1.4 Hz, 2 x H-C(9)), 7.68 - 7.65 (m, 4H, 2 x H-C(6) and 2 x H-C(7)), 7.41 (ddd, 2H, *J*(7,8) = 8.2 Hz, *J*(8,9) = 7.6 Hz, *J*(6,8) = 1.4 Hz, 2 x H-C(8)), 5.06 (s, 4H, 2 x NCH₂COOH);
¹³C-NMR (150 MHz, DMSO-*d*₆): *δ* 168.9 and 164.6 (2 x C-S and 2 x -COOH), 145.9, 142.9, 142.1, 132.1, 123.8, 122.3, 117.3, 112.1 and 42.9 (2 x -CH₂-).

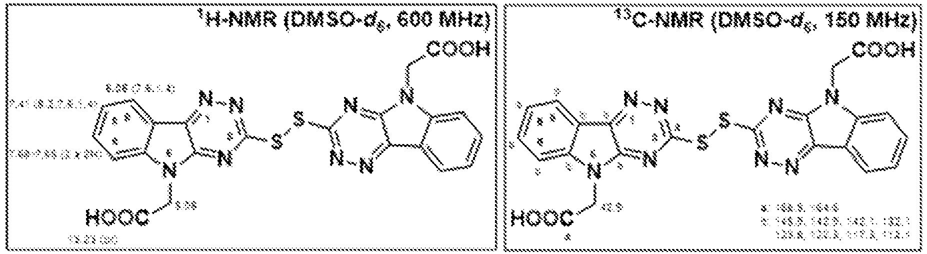

FT-IR (measured in solid state, cm⁻¹): 1730 (m), 1619 (w), 1577 (m), 1508 (w), 1467 (m), 1438 (m), 1413 (w), 1369 (m), 1334 (m), 1210 (m), 1174 (s), 1149 (m), 1089 (m), 1033 (w), 968 (m), 932 (m), 886 (w), 802 (m), 748 (s), 702 (w), 667 (m), 615 (m), 562 (w), 514 (w), 447 (m).

UV-Vis (DMSO): λₘₐₓ= 269 nm; HPLC-MS purity 98 %; Elemental analysis C₂₂H₁₄N₈O₄S₂ (518.526), calc.: C, 50.96; H, 2.72; N, 21.61 found: C, 50.87; H, 3.05; N, 21.80.

### Example 2

### Test A

### Reduction of disulfide of formula I by GSH

The cleavage kinetics of disulfide of formula **I** due to increasing concentrations of GSH to form cemtirestat of formula **II** was studied as follows: To a solution of disulfide of formula **I** (50 µM) in 20 mM phosphate buffer (pH 7.4) GSH was added in gradually increasing concentrations ranging from 0-100 µM. Immediately after the addition of GSH, the absorbance of the resulting cemtirestat of formula **II** was started at 302 nm. Cemtirestat of formula **II** concentration was calculated using an extinction coefficient ε = 38,4 mM⁻¹.cm⁻¹.

Compound of formula **I** of Example 1 was tested in Test A for the kinetics of reduction of disulfide of formula **I** to cemtirestat of formula **II** monomer in the presence of increasing concentrations of GSH and we found that dimer of compound of formula **I** splits in the presence of GSH to form the monomer (cemtirestat of formula **II**), so that with increasing concentrations of GSH, the rate and yield of the reaction increases (Fig. 1, Table 1).

**Table 1. Initial rate of cleavage of disulfide of formula I to form cemtirestat of formula II and final degree of reduction of disulfide of formula I in the presence of increasing concentrations of GSH.**

| GSH (µM) | 0 | 25 | 50 | 100 |
|---|---|---|---|---|
| Initial rate of cemtirestat of formula **II** formation (nmol/ml/s) | n.d. | 4.46 ± 1.68 | 7.30 ± 1.46 | 9.83 ± 2.09 |
| Final degree of disulfide of formula **I** reduction (%)* | n.d. | 39.68 ± 1.59 | 67.70 ± 3.72 | 85.16 ± 5.11 |

| | | | | |
|---|---|---|---|---|
| * Calculated after reaching the maximum in the 60th second. Disulfide of formula **I,** 50 µM; 20 mM phosphate buffer (pH 7.4) at 37 °C. Results are the arithmetic mean ± SD of three parallel experiments. Abbreviation n.d. means "undetectable low value". | | | | |

### Example 3

### Test B

### Inhibition of ALR2 by disulfide of formula I in the presence of GSH

*Preparation ofALR2.* The ALR2 enzyme from rat lenses was isolated by the method of Hayman and Kinoshit (J. Biol. Chem. 240: 877-882, 1965). The homogenate was centrifuged at 10,000 g at 0-4 °C for 20 min. The supernatant was precipitated with saturated ammonium sulfate solution gradually at 40 %, 50 % and finally at 75 % saturation. After the first two precipitations, the supernatant was used. The sediment from the last step, containing ALR2 activity, was resuspended in 75% ammonium sulfate and stored in smaller aliquots in a liquid nitrogen container.

*Enzyme test.* ALR2 activity was measured spectrophotometrically (Stefek et al.; Bioorg. Med Chem. 16: 4908-4920, 2008) by determining NADPH consumption at 340 nm and expressed as a decrease in optical density (O.D.) / s / mg protein. The reaction mixture contained 4.67 mM *D*,*L*-glyceraldehyde as substrate, 0.11 mM NADPH in 67 mM phosphate buffer (pH 7.4), GSH at the desired concentration and 0.05 ml of enzyme preparation a total volume of 1.5 ml. The reference (blank) sample contained all of the above components except *D*,*L*-glyceraldehyde substrate and was used to correct for NADPH oxidation unrelated to substrate reduction. Enzyme catalysis was initiated by the addition of *D*,*L*-glyceraldehyde and the reaction was monitored for 4 minutes after an initial 1 minute period at 37 °C. The enzyme activity was adjusted by diluting the enzyme preparations with distilled water so that 0.05 ml of the preparation gave an average reaction rate for the control samples in the range of 0.020 ± 0.005 absorbance units / min. The effect of inhibitor on enzyme activity was determined by adding a stock solution of inhibitor in DMSO to the reaction mixture at a final concentration of 100 µM so that the final DMSO concentration was 1%. An inhibitor with the same concentration was added to the reference sample. Enzyme activity was started 2 min after the addition of GSH to the reaction mixture by adding *D*,*L*-glyceraldehyde used as a substrate, and I(%) values were determined from the measured absorbance decrease values.

Compound of formula **I** of Example 1 was tested in Test B and was found to have I(%) values as a function of GSH concentration as shown in Table 2.

**Table 2. Inhibition of aldose reductase isolated from rat eye lenses by disulfide of formula I in the presence of increasing concentrations of GSH**

| GSH added (µM)* | 0 | 5 | 10 | 20 | 40 | 100 | 1000 | 5000 |
|---|---|---|---|---|---|---|---|---|
| Inhibition I (%) | < 1% | <1% | 8.9± 0.6 | 12.4± 1.4 | 16.1± 6.9 | 72.6± 9.1 | 94.0± 1.6 | 91.1± 1.5 |

| | | | | | | | | |
|---|---|---|---|---|---|---|---|---|
| * The natural GSH content of the ALR2 preparations used was <0.1 nmol/ml. Disulfide of formula **I** was used in the experiment at conc. of 100 µM; phosphate buffer had a concentration of 67 mM (pH 7.4) and was used at 37 °C. Enzyme activity was measured 2 min after the addition of GSH to the reaction mixture containing all components except *D*,*L*-glyceraldehyde used as substrate. Results are the arithmetic mean ± SD of three parallel experiments. | | | | | | | | |

### Example 4

### Test C

### Determination of the distribution ratio in the buffer/octan-1-ol system

The partition ratio in the buffer/1-octanol system (system name octan-1-ol), defined as the equilibrium ratio of the total solute concentration in the organic phase to the total solute concentration in the aqueous phase, was determined by the shake-flask method at room temperature. Octan-1-ol saturated with the desired buffer was used as the organic phase for the determination. Disulfide of formula **I** or cemtirestat of formula **II**, as a reference substance, was dissolved in the appropriate buffer (4 mL) at a final concentration of 100 µM in the presence of DMSO (1%). The aqueous solution was shaken with octan-1-ol (20 mL) for 3 hours. Subsequently, the phases were separated in a separatory funnel and the concentration of the solute was determined spectrophotometrically in both phases by means of a calibration curve.

The disulfide of formula **I** of Example 1 was tested in Test C and the distribution ratio in the phosphate buffer (pH 7.4) /octan-1-ol system was determined as shown in Table 3.

**Table 3. Distribution ratio of disulfide of formula I in the buffer/octan-1-ol system compared to cemtirestat of formula II**

| **Compound** | D | |
|---|---|---|
| | pH 7.4* | pH 4.6** |
| Disulfide of formula **I** | 0.0095 ± 0.0019 | 0.1136 ± 0.0022 |
| Cemtirestat of formula **II** | 0.0097 ± 0.0007 | 0.0353 ± 0.0007 |

| | | |
|---|---|---|
| *Phosphate buffer (0.1M; pH 7.4) + 0.15 M KCl; **Acetate buffer (0.1M; pH 4.6) + 0.15 M KCI; disulfide of formula **I** (100 µM); room temperature. Results are the arithmetic mean ± SD of three parallel experiments. | | |

### Industrial applicability

Industrial utility is that the compound of formula of formula **I** is a prodrug suitable for the production of a highly potent aldose reductase inhibitor of formula **II** with targeted distribution to cancer cells, with expected higher efficacy and fewer side effects than cemtirestat of formula **II** alone, but which is better absorbable in acidic tumor environments as cemtirestat of formula **II** alone.

## Claims

1. A compound cemtirestat disulfide which is 2,2'-(disulfanediylbis(5*H*-[1,2,4]triazino[5,6-*b*]indole-3,5-diyl))diacetic acid of formula **I:** 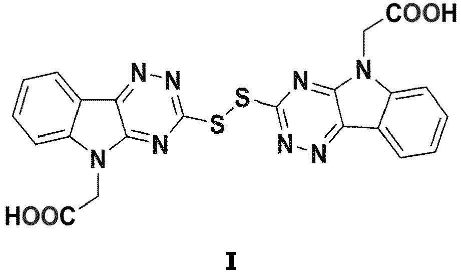 or a pharmaceutically acceptable form thereof selected from the group a mono- or dibasic salt, mono- or diesters, amides, or combinations thereof.

2. Process for preparation of the compound of formula **I**, according to claim 1, **characterized in that** it comprises the following steps:
- the cemtirestat compound of formula **II** 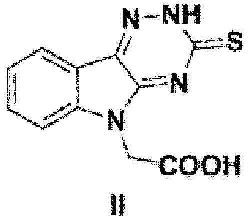
is treated with a mild oxidizing agent NaNO₂ in acetic acid at room temperature;
wherein the product of formula **I** precipitates from the reaction mixture already during the reaction;
- the product of formula **I** is filtered off and washed with ice water and cold methanol;
- the product of formula **I** is dried at a gradually reduced pressure from 3000Pa to 0.1Pa.

3. The compound of formula **I**, or a pharmaceutically acceptable form thereof according to claim 1, for use as a medicament.

4. The compound of formula **I**, or a pharmaceutically acceptable form thereof according to claim 1, for use in the treatment of conditions where inhibition of the aldo-keto reductases AKR1B1 and/or AKR1B10 in cells/tissues, possessing high concentration of reduced glutathione GSH, is desired.

5. The compound of formula **I** or a pharmaceutically acceptable form thereof according to claim 1, for use in the treatment of cancer originating from chronic inflammation, namely colon and rectal cancer, lung, breast, liver, pancreas, prostate, endometrium and cervical cancer.

6. The compound of formula **I**, or a pharmaceutically acceptable form thereof according to claim 1, for use in the treatment of cancer as an adjuvant therapeutic in combination with clinically used chemotherapeutics that are substrates of aldo-keto reductases such as doxorubicin and daunorubicin.

7. A pharmaceutical composition comprising as an active ingredient the compound cemtirestat disulfide of formula **I** or a pharmaceutically acceptable form thereof according to claim 1, in admixture with a pharmaceutically acceptable agent, diluent or carrier and optionally another excipient.

## Patentansprüche

1. Verbindung Cemtirestat Disulfid, die 2,2'-(Disulfandiylbis(5*H*-[1,2,4]triazino[5,6-b]indol-3,5-diyl))diessigsäure ist, der Formel **I:** 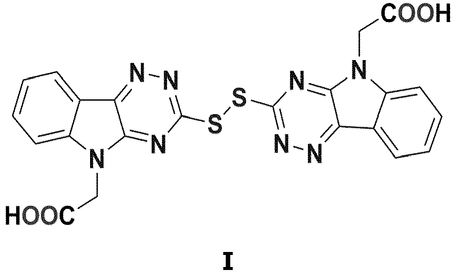 oder eine pharmazeutisch akzeptable Form davon, ausgewählt aus der Gruppe ein- oder zweibasisches Salz, Mono- oder Diester, Amide oder Kombinationen davon.

2. Verfahren zur Herstellung der Verbindung der Formel **I**, nach Anspruch 1, **dadurch gekennzeichnet, dass** es die folgenden Schritte umfasst:
- die Verbindung Cemtirestat der Formel **II** 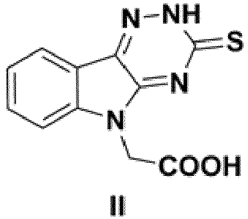
wird bei Raumtemperatur mit einem milden Oxidationsmittel NaNO₂ in Essigsäure behandelt;
wobei das Produkt der Formel **I** bereits während der Reaktion aus dem Reaktionsgemisch ausfällt;
- das Produkt der Formel **I** wird abfiltriert und mit Eiswasser und kaltem Methanol gewaschen;
- das Produkt der Formel **I** wird bei allmählich reduziertem Druck von 3000 Pa auf 0.1 Pa getrocknet.

3. Verbindung der Formel **I** oder eine pharmazeutisch akzeptable Form davon nach Anspruch 1 zur Verwendung als Medikament.

4. Verbindung der Formel **I** oder eine pharmazeutisch akzeptable Form davon nach Anspruch 1 zur Verwendung bei der Behandlung von Zuständen, bei denen die Inhibition der Aldo-Keto-Reduktasen AKR1B1 und/oder AKR1B10 in Zellen/Geweben, die eine hohe Konzentration an reduziertem Glutathion GSH besitzen, erwünscht ist.

5. Verbindung der Formel **I** oder eine pharmazeutisch akzeptable Form davon nach Anspruch 1 zur Verwendung bei der Behandlung von Krebs, der von einer chronischen Entzündung herrührt, nämlich Dickdarm- und Rektumkrebs, Lungen-, Brust-, Leber-, Pankreas-, Prostata-, Endometrium- und Gebärmutterhalskrebs.

6. Verbindung der Formel **I** oder eine pharmazeutisch akzeptable Form davon nach Anspruch 1 zur Verwendung bei der Behandlung von Krebs als adjuvantes Therapeutikum in Kombination mit klinisch verwendeten Chemotherapeutika, die Substrate von Aldo-Keto-Reduktasen sind, wie Doxorubicin und Daunorubicin.

7. Pharmazeutische Zusammensetzung, die als Wirkstoff die Verbindung Cemtirestat Disulfid der Formel **I** oder eine pharmazeutisch akzeptable Form davon nach Anspruch 1 enthält, im Gemisch mit einem pharmazeutisch akzeptablen Mittel, Verdünnungsmittel oder Träger und gegebenenfalls einem weiteren Hilfsstoff.

## Revendications

1. Un composé cemtirestat disulfide qui est un acide 2,2'-(disulfanediylbis(5*H-*[1,2,4]triazino[5,6-b]indole-3,5-diyl))diacétique avec formule **I** : 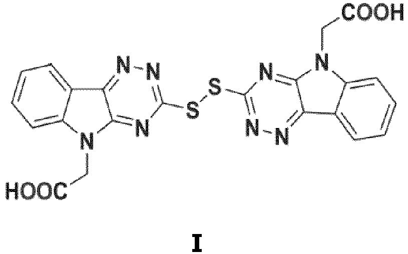 ou une forme pharmaceutiquement acceptable, choisie dans le groupe sel mono- ou dibasique, mono- ou diesters, amides ou combinaisons de ceux-ci.

2. Processus pour la préparation du composé de la formule **I**, selon la revendication 1, **caractérisé par le fait qu'**il comprend les étapes suivantes :
- le composé cemtirestat avec formule **II** 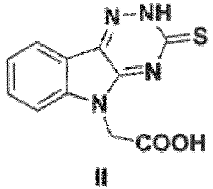
est traité avec un agent oxydant doux NaNO₂ dans de l'acide acétique à température ambiante ;
où le produit de formule **I** précipite du mélange réactionnel déjà pendant la réaction ;
- le produit formule **I** est filtré et lavé avec de l'eau glacée et du méthanol froid ;
- le produit formule **I** est séché sous une pression progressivement réduite de 3 000Pa à 0,1Pa.

3. Le composé formule **I** ou une forme pharmaceutiquement acceptable selon la revendication 1, pour utilisation comme médicament.

4. Le composé formule **I** ou une forme pharmaceutiquement acceptable selon la revendication 1, pour le traitement d'affections dans lesquelles on demande l'inhibition des aldo-céto réductases AKR1B1 et/ou AKR1B10 dans des cellules/tissues possédant une forte concentration de glutathion réduit GSH.

5. Le composé formule **I** ou une forme pharmaceutiquement acceptable selon la revendication 1, pour le traitement du cancer provenant d'une inflammation chronique, à savoir le cancer du côlon et du rectum, du poumon, du sein, du foie, du pancréas, de la prostate, de l'endomètre et du col de l'utérus.

6. Le composé formule **I** ou une forme pharmaceutiquement acceptable selon la revendication 1, pour le traitement du cancer comme adjuvant thérapeutique en combinaison avec les chimiothérapies utilisées en clinique qui sont des substrats des aldo-céto réductases telles que la doxorubicine et la daunorubicine.

7. Une composition pharmaceutique qui comprend, comme ingrédient actif, le composé cemtirestat disulfide formule **I** ou une forme pharmaceutiquement acceptable selon la revendication 1, en mélange avec un agent pharmaceutiquement acceptable, un diluant ou un vecteur et éventuellement un autre excipient.
